Europäisches Patentamt

European Patent Office

Office européen des brevets

(19)

(11) Publication number: 0 104 608
B1

# (12) EUROPEAN PATENT SPECIFICATION

(45) Date of publication of patent specification: 12.03.86

(21) Application number: 83109418.0

(22) Date of filing: 22.09.83

(51) Int. Cl.⁴: **C 12 N 11/00,** B 01 J 19/08, C 12 N 11/08, C 12 N 11/14, A 61 K 39/00, A 61 K 39/44

(54) **Chemically modified surface for large molecule attachment.**

(30) Priority: 24.09.82 US 422623

(43) Date of publication of application:
04.04.84 Bulletin 84/14

(45) Publication of the grant of the patent:
12.03.86 Bulletin 86/11

(84) Designated Contracting States:
BE DE FR GB IT NL SE

(56) References cited:
EP-A-0 046 613
EP-A-0 046 614
US-A-4 006 059
US-A-4 188 426

(73) Proprietor: **Becton, Dickinson and Company
Mack Centre Drive
Paramus New Jersey 07652 (US)**

(72) Inventor: **Dunn, Terry S.
1300 Canterbury Rd.
Raleigh North Carolina 27608 (US)**
Inventor: **Williams, Joel L.
1306 Walnut St.
Cary North Carolina 27511 (US)**

(74) Representative: **von Kreisler, Alek, Dipl.-Chem.
et al
Deichmannhaus am Hauptbahnhof
D-5000 Köln 1 (DE)**

EP 0 104 608 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates generally to methods and articles for influencing the attachment of large molecules to substrate surfaces. More particularly, the invention relates to the modification of the surfaces of organic and inorganic substrates so as to provide a chemically modified surface which influences the orientation and attachment of large molecules having available functional groups, such as proteins, to substrate surfaces.

It is demonstrated herein that the orientation and attachment of large molecules on polymer surfaces can be selectively controlled by varying the surface chemistry of the substrate in contact with the large molecule.

In a preferred embodiment of the present invention, the surface chemistry of a substrate is modified by subjecting the substrate to a plasma of a suitable material. It is known to subject substrates to plasmas of various types for accomplishing various purposes. A brochure of Tegal Corporation, CP1287, for example indicates that plasmas can be used for dry stripping, cleaning and etching of electronic parts, cleaning optical surfaces, preparing surfaces having bonding, wettability and chemical resistance properties and dry cleaning of substrates to improve film deposition and adherence. It is not known, however, that the attachment and orientation of large molecules can be influenced by chemical modification of the surface of the substrate on which the molecule is attached.

The importance of the ability to control the attachment of large molecules (either to enhance or hinder such attachment) is self evident. The importance of being able to control the orientation of attachment can be illustrated by reference to a particular large molecule, such as an antibody. Antibodies are large protein molecules having a Y configuration. The two short arms of the Y ($F_{ab}$) contain the immunologically active sites which are capable of recognizing an antigen. For some immunological assays it is desirable to couple the antibody to a substrate. Such coupling must take place predominantly by attachment of the long arm of the Y ($F_c$) antibody configuration to retain the immunologic activity of the antibody.

Other examples of large molecules having available functional groups which are sometimes attached to a substrate wherein control of attachment and orientation are important include: proteins bound to a column for purification of other proteins by column elutriation, immobilization of enzymes in a biologically active mode and binding of hormones for biological treatment.

Accordingly, it is a principal object of the present invention to provide chemically specific surfaces for influencing the attachment and/or orientation of large molecules having available functional groups onto the surface.

It is another object of the present invention to provide a substrate with a chemically modified surface which influences the attachment and/or orientation of large molecules onto the surface.

It is a further object of the present invention to provide a substrate with a chemically modified surface controls the attachment and orientation of antibodies onto the substrate surface.

It is a still further object of the present invention to provide a method and an article for controlling the attachment and/or orientation of biologically active large molecules having available functional groups onto the surface of the article.

These and other objects of the present invention will become more apparent from the accompanying drawings wherein:

Fig. 1 is a schematic diagram of an apparatus which can be employed in the practice of the invention.

Fig. 2 is a perspective view of a sample holder illustrated in the apparatus of Fig. 1.

Figure 3 is a schematic representation of an antibody.

Figs. 4 through 6 are plots of the attachment and orientation characteristics of various untreated substrates and substrates treated in accordance with the invention.

In general, it has been discovered that the attachment and/or orientation of biologically active large molecules can be controlled by varying the surface chemistry of a substrate surface in contact with the large molecule. The surface chemistry of a polymeric surface can be varied by using selected homopolymers, copolymerization, surface grafting and chemical modification, and by plasma treatment of the surface. In particular, plasma modification processes may be used to provide specific chemical functional groups, elemental ratios and other surface properties. In accordance with the invention, polymeric surfaces were prepared by plasma modification techniques which yielded a range of surface chemistries and properties. The modified polymeric surfaces were subjected to solutions of biologically active large molecules and subsequently tested to demonstrate that attachment and orientation of the large molecule is highly dependent on surface chemistry.

As used herein, the term "large molecule" means any organic or inorganic molecule having a molecular weight greater than about 1,000 g/molecular. The termm "available functional group" means a chemically active functional group contained in a large molecule which is available for binding to a substrate. Suitable functional groups include amino, carboxyl, sulfhydryl, hydroxyl, sulfate, alkylhalides, to name a few. The term "biologically active large molecule" means an amino acid containing molecule (usually a protein) which has biological activity.

A plasma of a suitable material may be used to modify the surface layer of organic and inorganic substrates, including polymeric materials, glass and metals, regardless of thickness and which is independent of substrate composition, so as to provide specific chemical functional groups on the surface of the substrate. In the case of glass and metals, it is desirable to include a carbon source, such as methane, for example, to provide a thin organic layer on the organic substrate. Such plasma process is suitable for providing a surface for any cellular growth application whether the desired goal is to enhance or retard cell growth. The surface of the substrate is irreversibly modified by grafting specific chemical functional groups onto the surface with a plasma of a suitable material or with selected components of a plasma of a suitable material. Suitable materials include, but are not limited to oxygen, carbon, hydrogen, nitrogen, halogen, sulfur, phosphorus, mixtures thereof and compounds thereof. The surfaces which are modified in accordance with the present invention have specific chemical functional groups grafted onto the substrate surface and provide suitable surface chemistry for tissue culture growth or suppression of growth.

In another embodiment of the present invention there is provided a process for modifying the surface of a substrate, specifically the modification of organic polymeric materials by treatment with specific chemical species comprising:

Providing a substrate within a reaction zone.

Providing a plasma of a suitable material in the reaction zone which includes neutral material, positive ions of the material, negative ions of the material, electrons and photons.

Preventing at least one of the components of the plasma from contacting the surface of the substrate in the reaction zone.

Contacting the surface of the substrate with the remainder of the components of the plasma in the reaction zone, and

Forming specific functional groups of the material on the surface of the substrate.

This embodiment of the present invention is based on the discovery that utilization of selected reactive species of a plasma in contact with a substrate can be employed to provide specific chemical functional groups on the surface of the substrate which act to influence the attachment and/or orientation of large molecules.

Referring now to the figures, other features and advantages of the present invention will be described.

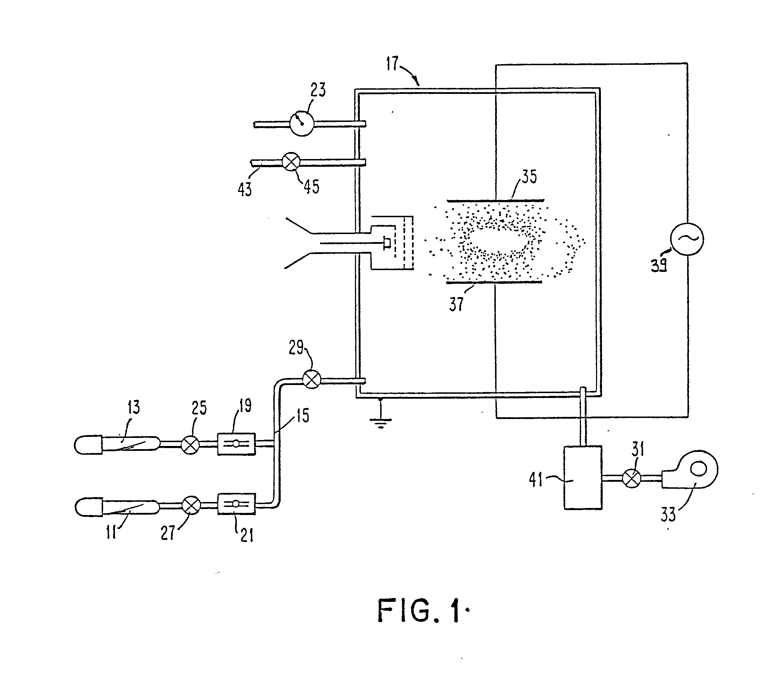

As shown in Fig. 1 there is provided a first gas reservoir 11, a second gas reservoir 13 with conduit means 15 to deliver either or both a first gas and a second gas to a vacuum chamber 17. Flow meters 19 and 21 are provided for measuring gas flow rate and a vacuum gauge 23 is provided to monitor the pressure within the vacuum chamber 17. Valves 25, 27 and 29 are provided to regulate the flow rate of the gas in the first reservoir 11, the second gas reservoir 15 and the gas entering the vacuum chamber 17. Prior to use, the vacuum chamber 17 is evacuated by opening valve 31 to vacuum pump 33. Suitable electrodes 35 and 37 are connected to a suitable voltage source 39. The reactor system also includes a trap 41, vent conduit 43 and its valve 45.

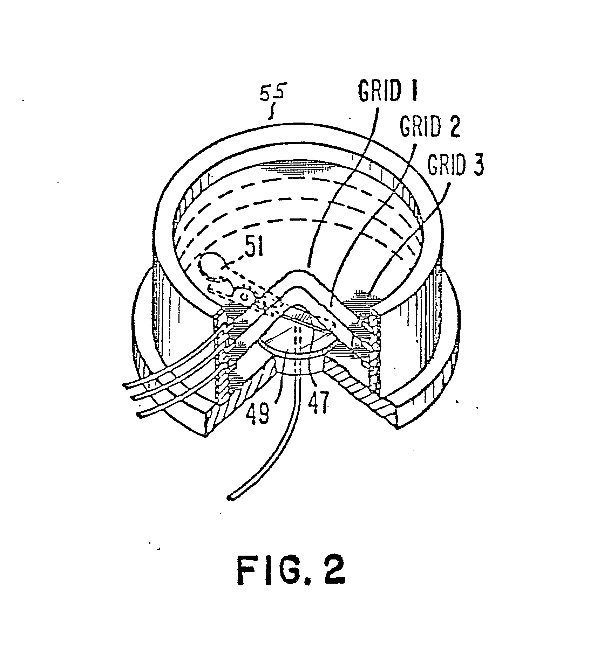

As best seen in Fig. 2, a substrate 47 to be treated in accordance with the invention is placed in a sample holder 55 and disposed within the vacuum chamber 17. The sample holder includes a grid assembly of three grids, designated grid 1, grid 2 and grid 3. A collector 49 which can be electrically biased is disposed on the other side of the sample from the grid system. The sample is held in place between grid 3 and the collector by means of a suitable holder 51.

In operation, any or none of the three grids or the collector can be biased with positive or negative charge to repel selective components of a plasma generated between the electrodes 35 and 37. The substrate in the sample holder is then subjected to reaction with those components of the plasma which are not repelled.

The present invention can be employed to alter the surfaces of solid polymeric materials, including natural and synthetic addition and condensation polymers. Such polymeric materials include polyolefins, such as polyethylene, polypropylene, polyisobutylene and ethylene-alphaolefin copolymers, arcylic polymers and copolymers, such as polyacrylate, polyethylmethacrylate, polyethylacrylate; vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl amines; polyvinyl aromatics, such as polystyrene, polyvinyl esters such as polyvinyl acetate; copolymers of vinyl monomers with each other and olefins, such as ethylene-methyl methacrylate copolymers, acrylonitrile-styrene copolymers, ABS resins, and ethylenevinyl acetate copolyers; natural and synthetic rubbers, including butadiene-styrene copolymers, polyisoprene, synthetic polyisoprene, polybutadiene, butadiene-acrylonitrile copolymers, polychloroprene rubbers, polyisobutylene rubber, ethylene-propylene rubber, ethylene-propylene-diene rubbers, isobutylene-isoprene copolymers and polyurethane rubbers; polyamides, such as Nylon 66 and polycaprolactam; polyesters such as polyethylene terephthalate, alkyd resins; phenol-formaldehyde resins; urea-formaldehyde resins, malamine-formaldehyde resins; polycarbonates; polyoxymethylenes; polyimides; polyethers, epoxy resins, poly-urethanes; wool; cotton; silk; rayon; rayon-triacetate; cellulose; cellulose acetate, cellulose butyrate; cellulose acetatebutyrate; cellophane; cellulose nitrate; cellulose propionate; cellulose ethers; and carboxymethyl cellulose.

Inorganic materials, the surfaces of which can be modified in accordance with the invention, include non-metals, such as graphite; metals, such as iron, aluminum, tin, copper and nickel; metal and other elemental oxides; such as magnesium oxide, silica, alumina and titania; minerals, such as clay, pyrite and asbestos; salts, such as sodium chloride and calcium carbonate; and such synthetic compositions such as glass and refactories.

The substrates whether organic or inorganic, can be any shape, such as continuous or particulate, porous or impervious, and large or small. The invention can be employed for altering the surfaces of crystals, powders, plates, strips, films, sheets, wire, fibers, fabrics, filaments, tubing, and cast, extruded or compressed articles, and the like.

Plasmas of the elements of carbon, hydrogen, oxygen, nitrogen, sulfur, phosphorus, the halogens, mixtures thereof and compounds thereof are useful to modify the surface of the substrates in accordance with the present invention.

Four types of plasmas can be generated; these are thermal plasmas, discharge plasmas, beam plasmas (such as ion beams and electron beams) and hybrid plasmas such as corona discharges. Suitable plasmas for use in the method of the present invention are discharge plasmas, beam plasmas and hybrid plasmas. Suitable plasmas can be generated by use of DC sources or AC sources having a frequency through the microwave range at power levels from about 1.0 W to about 10 kW. Although not limited to high frequency sources, such as radiofrequency (r.f.) sources, plasmas of this type are particularly useful for the method and articles of the present invention in that selected plasma species concentrations can be controlled by source frequency, power, and system pressure. It is also true that r.f. plasmas are suitable for uniformly modifying the surface of more complex article shapes, such as tubing and other enclosures, for which DC and low frequency generated plasmas are not satisfactory.

In general, the r.f. plasmas suitable for use in the present invention are generated at a frequency of from about 1.0 to about 300 MHz at a power to initiate breakdown, such as from about 5 to about 1000 watts at pressures ranging from 0.001 to 10 Torr. The articles are usually subjected to the r.f. plasma for a period of from about 0.1 sec to about 120 minutes, although longer and shorter periods can be used. The plasma treatment can be followed by a quench cycle designed to provide an atmosphere suitable for reacting with residual active polymer species at or near the surface with pressures ranging from 1 Torr to 760 Torr for time periods of 1 second to 4 hours. The r.f. plasma can be generated between two flat plates, as shown in Figure 1, or can be generated with a helical coil, as shown in United States Patent No. 4,188,426.

The following examples further illustrate various features of the invention but are not intended to in any way limit the scope of the invention which is set forth in the appended claims.

Example I

It is well known to test for small concentrations of antigens using antibodies and radiolabeling techniques. This technique is generally referred to as RIA (radioimmunoassay) and is widely used commercially. A specialized form of RIA referred to as solid phase RIA relies on the ability of a polymer to bind the antibody. When an antigen is brought into proximity to the polymer, the antigen reacts with the bound antibody. A competitive assay can be developed by adding a known amount of labeled antigen to the tube. The method, however, is highly dependent on the immunologic activity of the bound antibody maintained on the polymer surface.

In the present invention, a method is disclosed that allows control of the surface concentration of antibody binding and the orientation of the antibody molecule on the substrate surface by plasma surface modification processes.

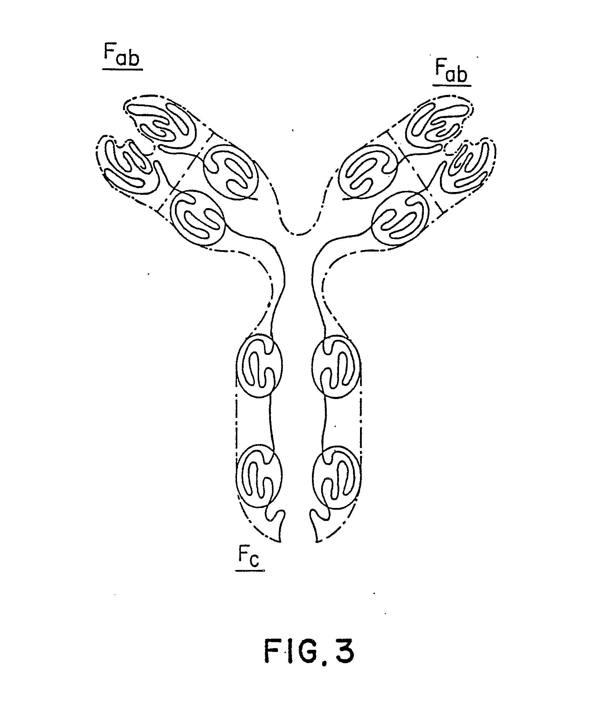

The chemical definitions of the IgG antibody has evolved to designate the $F_c$ fragment and $F_{ab}$ fragments separately. The immunologically active sites of the antibody reside in the $F_{ab}$ fragments as illustrated in the attached diagram of the IgG molecule contained in Figure 3. If an antibody is coated onto a surface, the $F_{ab}$ fragments must be accessible for binding antigen. Therefore, the preferred orientation for binding IgG to a solid substrate is with the $F_c$ fragment attached to the substrate leaving the two $F_{ab}$ fragments accessible for binding.

Polystyrene molded strips were placed in the vacuum chamber of the apparatus of Fig. 1. The vacuum chamber 17 was subsequently evacuated to 50 μ for 20 minutes and then filled to 200 μ with $SO_2$ for 10 minutes. A 10 MHz r.f. plasma was operated for five minutes with 45 watts power. The vacuum chamber 17 was maintained at 200 μm of $SO_2$ for two minutes, pumped down to 50 μ for two minutes, and then exposed to air at atmospheric pressure. The relative zeta potential of the surface of the polystyrene strips was determined to be −19.4 mV. The surface tension as measured by Wilhelmy plate contact angle was determined to be 72°±3°.

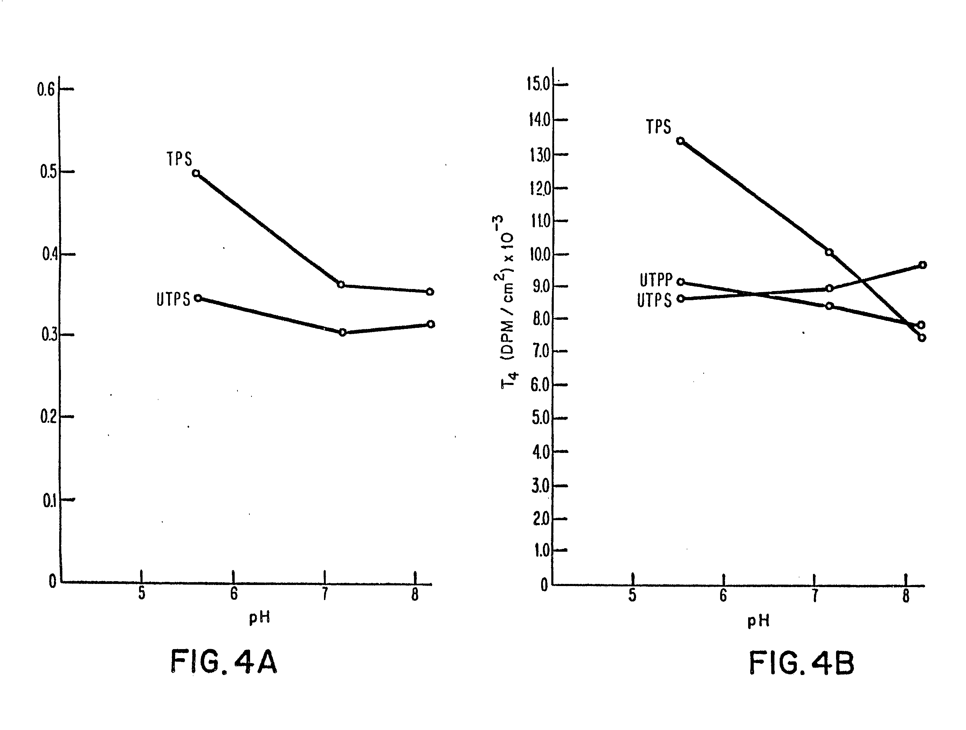

To measure antibody binding to the modified surface, purified (ammonium sulfate fractionation) rabbit IgG was radiolabeled with [125]I. The amount of IgG bound was measured at ambient temperature as a function of pH of the coating solution comprised of 10 μg/ml IgG in phosphate buffered saline with ionic strength of 0.156 M. The surface of polystyrene strips (both treated and untreated by the plasma process described above) was exposed to the solution for 60 minutes and centrifuged dry. The specific activity of the labeled IgG was $2.2 \times 10^3$ DPM/μg. The results of the antibody binding experiment are illustrated in Figure 4a. At low pH the amount of antibody bound has increased by 50%.

For the measurement of immunologic activity to $T_4$ the surface was exposed to a solution of $T_4$-antiserum purified by affinity column chromatography. The $T_4$-antiserum solution concentration used was 100 μg/ml in phosphate buffered saline with ionic strength of 0.156 M. The surface of the polystyrene strips were exposed to the antiserum solution for 20 minutes and centrifuged dry. The coated surface was then exposed to radiolabeled $T_4$ solution for 45 minutes at room temperature and rinsed under running water.

The results of the $T_4$ measurement are given in Figure 4b. At low pH the amount of antigen bound to plasma treated polystyrene strip was increased by 55% as compared to the untreated polystyrene strip.

Example 2

Polystyrene molded strips were placed into the vacuum chamber 17 which was evacuated to 75μ for 10 minutes and then brought up to a pressure of 180μ of $NH_3$ per a period of two minutes. A 10 MHz r.f. plasma was operated for five minutes at a power of 35 watts. The chamber was then exposed to air at atmospheric pressure. The measured relative zeta potential was +8.2 mV. The Wilhelmy plate contact angle was determined to be 60°±2°.

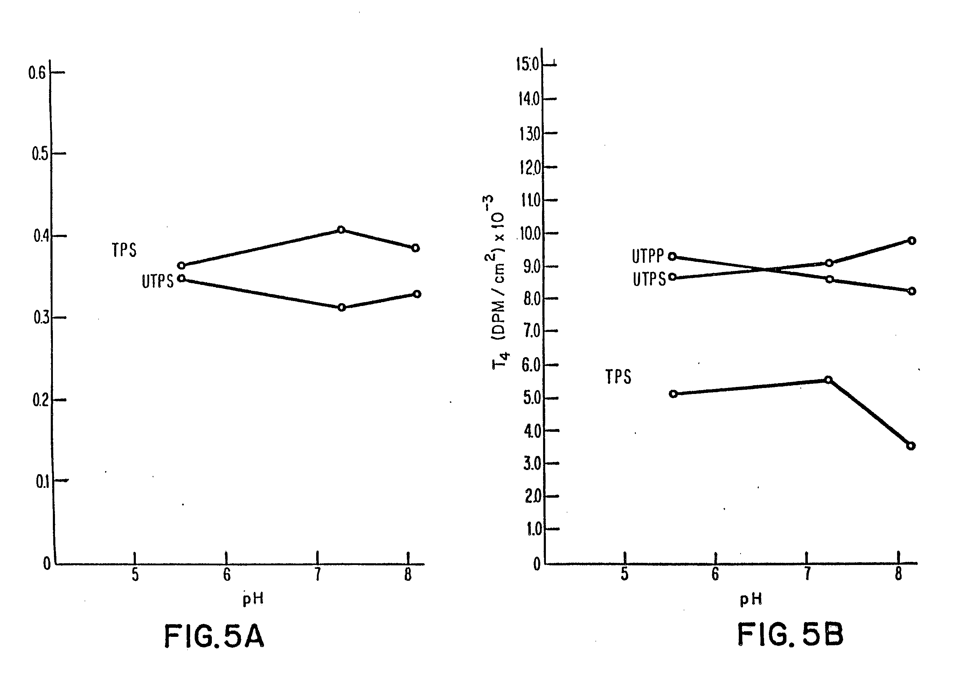

To measure antibody binding to the modified surface, purified (ammonium sulfate fractionation) rabbit IgG was radiolabeled with [125]I. The amount of IgG bound was measured at ambient temperature as a function of pH of the coating solution comprised of 10 μg/ml IgG in phosphate buffered saline with ionic strength of 0.156 M. The surface of treated and untreated polystryene strips were exposed to the solution for 60 minutes and centrifuged dry. The specific activity of the labeled IgG was $2.2 \times 10^3$ DPM/μg. The results of the antibody binding experiment are illustrated in Figure 5a. The maximum increase in the amount of antibody bound at any pH is 25%.

For the measurement of immunologic activity to $T_4$ the surface was exposed to a solution of $T_4$-antiserum purified by affinity column chromatography. The $T_4$-antiserum solution concentration used was 100 μg/ml in phosphate buffered saline with ionic strength of 0.56 M. The surface of the polystyrene strips were exposed to the antiserum solution for 20 minutes and centrifuged dry. The coated surface was then exposed to radiolabeled $T_4$ solution for 45 minutes at room temperature and rinsed under running water.

The results of the $T_4$ study are given in Figure 5b. It is evident from the data that there is a significant decrease in $T_4$ bound to the plasma treated polystyrene strips even though an increase in antibody binding was measured. This data indicates that fewer $F_{ab}$ sites are exposed and illustrates that the orientation of the IgG has been significantly affected by the surface treatment.

Example 3

Polystyrene molded strips were placed into a vacuum chamber 17 which was evacuated to 75μ for 10 minutes and then brought up to a pressure of 180μ of $NH_3$ for a period of two minutes. A 10 MHz r.f. plasma was operated for five minutes at a power of 35 watts. The chamber was then brought up to a 700 Torr pressure of $CO_2$ for a two-hour quench cycle. The plasma chamber was pumped down to 1 Torr and then opened to air at atmospheric pressure. The relative zeta potential of this surface was measured as +31.3 mV. The Wilhelmy plate contact angle was determined to be 64°±3°.

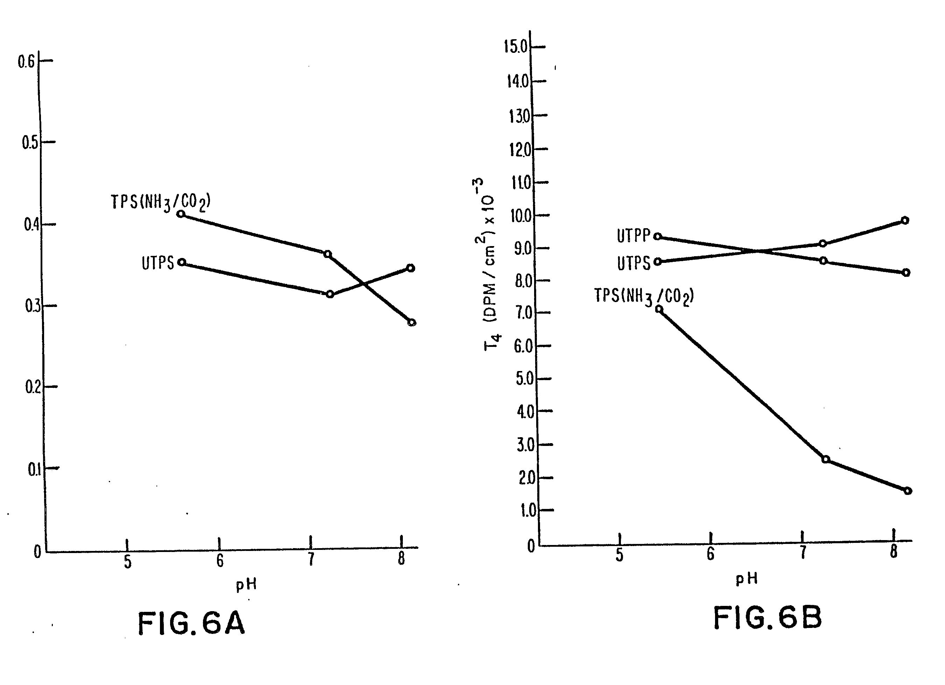

To measure antibody binding to the modified surface, purified (ammonium sulfate fractionation) rabbit IgG was radiolabeled with [125]I. The amount of IgG bound was measured at ambient temperature as a function of pH of the coating solution comprised of 10 μg/ml IgG in phosphate buffered saline with ionic strength of 0.156 M. The surface of treated and untreated polystyrene strips was exposed to the solution for 60 minutes and centrifuged dry. The specific activity of the labeled IgG was $2.2 \times 10^3$ DPM/μg. The results of the antibody binding experiment are illustrated in Figure 6a. The maximum increase in IgG bound to the surface at any pH is 20%

For the measurement of immunologic activity to $T_4$ the surface was exposed to a solution of $T_4$-antiserum purified by affinity column chromatography. The $T_4$-antiserum solution concentration used was 100 μg/ml in phosphate buffered saline with ionic strength of 0.156 M. The surface of the polystyrene strips was exposed to the antiserum solution for 20 minutes and centrifuged dry. The coated surface was then exposed to radiolabeled $T_4$ solution for 45 minutes at room temperature and rinsed under running water.

The results of the $T_4$ study are given in Figure 6b. It is evident from the data that there is a significant decrease in $T_4$ bound to the plasma treated polystyrene strips even though an increase in IgG antibody binding was measured. This data further substantiates that the orientation of the IgG antibody molecule has been significantly affected by the surface treatment.

Claims

1. A method for influencing the attachment and/or orientation of large molecules on a substrate comprising modifying the surface chemistry of said substrate by subjecting the surface of said substrate to a plasma which is produced from a material selected from the group consisting of carbon, hydrogen, oxygen, nitrogen, sulfur, phosphorous, halogens and compounds thereof.

2. A method in accordance with Claim 1 wherein said plasma is a discharge plasma generated at a frequency of from about 1 to about 300 megahertz at a power of from about 5 to about 1000 watts at a pressure of from about 0.001 to about 10 Torr.

3. A method in accordance with Claims 1 and 2 wherein said plasma material is selected from oxygen and oxygen compounds.

4. A method in accordance with Claims 1 and 2 wherein said plasma material is selected from nitrogen and nitrogen compounds.

5. A method in accordance with Claims 1 and 2 wherein said plasma material is selected from sulfur and sulfur compounds.

6. A method in accordance with Claims 1 and 2 wherein said plasma material is selected from phosphorus and phosphorus compounds.

7. A method in accordance with Claims 1 and 2 wherein said plasma material is selected from halogen and halogen compounds.

8. A method in accordance with Claims 1 and 2 wherein said plasma material is selected from carbon and carbon compounds.

9. A method in accordance with Claims 1 and 2 wherein said plasma is selected from hydrogen and hydrogen compounds.

10. A method in accordance with Claim 1 wherein said large molecule is a protein.

11. A method in accordance with Claim 1 wherein said large molecule is an antibody.

12. A method in accordance with Claim 1 wherein said large molecule is an antigen.

13. An article suitable for influencing the attachment and/or orientation of large molecules thereto comprising a polymeric article whose surface chemistry has been modified by subjecting the surface of said polymeric article to a plasma which is produced from a material selected from the group consisting of carbon, hydrogen, oxygen, nitrogen, sulfur, phosphorus, halogens and compounds thereof, wherein plasma is a discharge plasma generated at a frequency of from about 1 to about 300 megahertz at a power of from about 5 to about 1000 watts at a pressure of from about 0.001 to about 10 Torr. characterised in that the plasma material is selected from oxygen and oxygen compounds.

14. An article in accordance with Claim 13 wherein said plasma material is selected from nitrogen and nitrogen compounds.

15. An article in accordance with Claim 13 wherein said plasma material is selected from sulfur and sulfur compounds.

16. An article in accordance with Claim 13 wherein said plasma material is selected from phosphorus and phosphorus compounds.

17. An article in accordance with Claim 13 wherein said plasma material is selected from hydrogen and hydrogen compounds.

18. An article in accordance with Claim 13 wherein said large molecule is a protein.

19. An article in accordance with Claim 13 wherein said large molecule is an antibody.

20. An article in accordance with Claim 13 wherein said large molecules is an antigen.

21. An article suitable for influencing the attachment and/or orientation of large molecules thereto comprising an inorganic article whose surface chemistry has been modified by subjecting the surface of said inorganic article to a plasma which is produced from a material selected from the group consisting of carbon, hydrogen, oxygen, nitrogen, sulfur, phosphorus, halogens and compounds thereof, wherein plasma is a discharge plasma generated at a frequency of from about 1 to about 300 megahertz at a power of from about 5 to about 1000 watts at a pressure of from about 0.001 to about 10 Torr, characterised in that plasma material is selected from oxygen and oxygen compounds.

22. An article in accordance with Claim 21 wherein said plasma material is selected from nitrogen and nitrogen compounds.

23. An article in accordance with Claim 21 wherein said plasma material is selected from sulfur and sulfur compounds.

24. An article in accordance with Claim 21 wherein said plasma material is selected from phosphorus and phosphorus compounds.

25. A method for influencing the attachment and/or orientation of large molecules on a substrate comprising modifying the surface chemistry of said substrate by a method comprising;

(a) providing said substrate within a reaction zone

(b) introducing a material into said reaction zone, said material being suitable for providing a plasma

(c) subjecting said material to plasma producing conditions, whereby the plasma comprises neutral material, positive ions of said material, negative ions of said material, electrons and photons

(d) preventing at least one of the components of said plasma from contacting the surface of said substrate,

(e) contacting the surface of said substrate with the remainder of said components of said plasma, and

(f) forming specific functional groups of said material on the surface of said organic substrate.

26. A method in accordance with Claim 25 wherein said plasma is produced under electrical discharge conditions.

27. A method in accordance with Claims 25 and 26 wherein said material is selected from oxygen, nitrogen, halogen, sulfur, compounds thereof and mixtures thereof.

28. A method in accordance with Claim 25 and 26 wherein said vaporized material is oxygen and compounds thereof.

29. A method in accordance with Claims 25 and 26 wherein said vaporized material is nitrogen and compounds thereof.

30. A method in accordance with Claims 25 and 26 wherein said vaporized material is halogen and compounds thereof.

31. A method in accordance with Claims 25 and 26 wherein said vaporized material is sulfur and compounds thereof.

32. A method in accordance with Claim 25 wherein said large molecule is a protein.

33. A method in accordance with Claim 25 wherein said large molecule is an antibody.

34. A method in accordance with Claim 25 wherein said large molecule is an antigen.

**Patentansprüche**

1. Verfahren zur Beeinflussung der Haftung und/oder Orientierung großer Moleküle auf einem Substrat durch Modifizieren der Oberflächenchemie des Substrats in der Weise, daß die Oberfläche des Substrats einem Plasma ausgesetzt wird, das aus einem aus der aus Kohlenstoff. Wasserstoff, Sauerstoff, Stickstoff, Schwefel, Phosphor, Halogenen und deren Verbindungen bestehenden Gruppe ausgewählten Material erzeugt wird.

2. Verfahren nach Anspruch 1, dadurch gekennzeichnet, daß das Plasma ein Entladungs-Plasma ist, das bei einer Frequenz von etwa 1 bis etwa 300 MHz bei einer Leistung von etwa 5 bis etwa 1000 W bei einem Druck von etwa 0,001 bis etwa 10 Torr erzeugt wird.

3. Verfahren nach Ansprüchen 1 und 2, dadurch gekennzeichnet, daß das Plasma-Material aus Sauerstoff und Sauerstoff-Verbindungen ausgewählt ist.

4. Verfahren nach Ansprüchen 1 und 2, dadurch gekennzeichnet, daß das Plasma-Material aus Stickstoff und Stickstoff-Verbindungen ausgewählt ist.

5. Verfahren nach Ansprüchen 1 und 2, dadurch gekennzeichnet, daß das Plasma-Material aus Schwefel und Schwefel-Verbindungen ausgewählt ist.

6. Verfahren nach Ansprüchen 1 und 2, dadurch gekennzeichnet, daß das Plasma-Material aus Phosphor und Phosphor-Verbindungen ausgewählt ist.

7. Verfahren nach Ansprüchen 1 und 2, dadurch gekennzeichnet, daß das Plasma-Material aus Halogenen und Halogen-Verbindungen ausgewählt ist.

8. Verfahren nach Ansprüche 1 und 2, dadurch gekennzeichnet, daß das Plasma-Material aus Kohlenstoff und Kohlenstoff-Verbindungen ausgewählt ist.

9. Verfahren nach Ansprüchen 1 und 2, dadurch gekennzeichnet, daß das Plasma-Material aus Wasserstoff und Wasserstoff-Verbindungen ausgewählt ist.

10. Verfahren nach Anspruch 1, dadurch gekennzeichnet, daß das große Molekül ein Protein ist.

11. Verfahren nach Anspruch 1, dadurch gekennzeichnet, daß das große Molekül ein Antikörper ist.

12. Verfahren nach Anspruch 1, dadurch gekennzeichnet, daß das große Molekül ein Antigen ist.

13. Gegenstand, der sich für die Beeinflussung der Haftung und/oder Orientierung großer Moleküle auf demselben eignet, umfassend einen polymeren Gegenstand, dessen Oberflächenchemie in der Weise modifiziert ist, daß die Oberfläche des polymeren Gegenstandes einem Plasma ausgesetzt wird, das aus einem aus der aus Kohlenstoff. Wasserstoff, Sauerstoff, Stickstoff, Schwefel, Phosphor, Halogenen und deren Verbindungen bestehenden Gruppe ausgewählten Material erzeugt wird, wobei das Plasma ein Entladungs-Plasma ist, das bei einer Frequenz von etwa 1 bis etwa 300 MHz bei einer Leistung von etwa 5 bis etwa 1000 W bei einem Druck von etwa 0,001 bis etwa 10 Torr erzeugt wird, dadurch gekennzeichnet, daß das Plasma-Material aus Sauerstoff und Sauerstoff-Verbindungen ausgewählt ist.

14. Gegenstand nach Anspruch 13 dadurch gekennzeichnet, daß das Plasma-Material aus Stickstoff und Stickstoff-Verbindungen ausgewählt ist.

15. Gegenstand nach Anspruch 13 dadurch gekennzeichnet, daß das Plasma-Material aus Schwefel und Schwefel-Verbindungen ausgewählt ist.

16. Gegenstand nach Anspruch 13 dadurch gekennzeichnet, daß das Plasma-Material aus Phosphor und Phosphor-Verbindungen ausgewählt ist.

17. Gegenstand nach Anspruch 13 dadurch gekennzeichnet, daß das Plasma-Material aus Wasserstoff und Wasserstoff-Verbindungen ausgewählt ist.

18. Gegenstand nach Anspruch 13, dadurch gekennzeichnet, daß das große Molekül ein Protein ist.

19. Gegenstand nach Anspruch 13, dadurch gekennzeichnet, daß das große Molekül ein Antikörper ist.

20. Gegenstand nach Anspruch 13, dadurch gekennzeichnet, daß das große Molekül ein Antigen ist.

21. Gegenstand, der sich für die Beeinflussung der Haftung und/oder Orientierung großer Moleküle auf demselben eignet, umfassend einen anorganischen Gegenstand, dessen Oberflächenchemie in der Weise modifiziert ist, daß die Oberfläche des polymeren Gegenstandes einen Plasma ausgesetzt wird, das aus einem der aus Kohlenstoff. Wasserstoff, Sauerstoff, Stickstoff, Schwefel, Phosphor, Halogenen und deren Verbindungen bestehenden Gruppe ausgewählten Material erzeugt wird, wobei das Plasma ein Entladungs-Plasma ist, das bei einer Frequenz von etwa 1 bis etwa 300 MHz bei einer Leistung von etwa 5 bis etwa 1000 W bei einem Druck von etwa 0,001 bis etwa 10 Torr erzeugt wird, dadurch gekennzeichnet, daß das Plasma-Material aus Sauerstoff und Sauerstoff-Verbindungen ausgewählt ist.

22. Gegenstand nach Anspruch 21 dadurch gekennzeichnet, daß das Plasma-Material aus Stickstoff und Stickstoff-Verbindungen ausgewählt ist.

23. Gegenstand nach Anspruch 21 dadurch gekennzeichnet, daß das Plasma-Material aus Schwefel und Schwefel-Verbindungen ausgewählt ist.

24. Gegenstand nach Anspruch 21 dadurch gekennzeichnet, daß das Plasma-Material aus Phosphor und Phosphor-Verbindungen ausgewählt ist.

25. Verfahren zur Beeinflussung der Haftung und/oder Orientierung großer Moleküle auf einen Substrat durch Modifizieren der Oberflächenchemie des Substrats mittels eines Verfahrens, in dem

(a) das Substrat in das Innere einer Reaktionszone gebracht wird,

(b) ein Material, das sich für die Erzeugung eines Plasmas eignet, in die Reaktionszone gebracht wird,

(c) das Material Bedingungen unterworfen wird, unter denen ein Plasma erzeugt wird, wobei dieses Plasma das neutrale Material, positive lonen des Materials, negative lonen des Materials, Elektronen und Photonen umfaßt,

(d) der Kontakt wenigstens einer der Komponenten des Plasmas mit der Oberfläche verhindert wird,

(e) die Oberfläche mit dem Rest der Komponenten des Plasmas in Kontakt gebracht wird und

(f) spezifische funktionelle Gruppen des Materials auf der Oberfläche des organischen Substrats gebildet werden.

26. Verfahren nach Anspruch 25, dadurch gekennzeichnet, daß das Plasma unter elektrischen Entladungsbedingungen erzeugt wird.

27. Verfahren nach Ansprüchen 25 und 26, dadurch gekennzeichnet, daß das Material aus Sauerstoff, Stickstoff, Halogenen, Schwefel, deren Verbindungen und Mischungen daraus ausgewählt ist.

28. Verfahren nach Ansprüchen 25 und 26, dadurch gekennzeichnet, daß das verdampfte Material aus Sauerstoff oder dessen Verbindungen besteht.

29. Verfahren nach Ansprüchen 25 und 26, dadurch gekennzeichnet, daß das verdampfte Material aus Stickstoff oder dessen Verbindungen besteht.

30. Verfahren nach Ansprüchen 25 und 26, dadurch gekennzeichnet, daß das verdampfte Material aus Halogenen oder deren Verbindungen besteht.

31. Verfahren nach Ansprüchen 25 und 26, dadurch gekennzeichnet, daß das verdampfte Material aus Schwefel oder dessen Verbindungen besteht.

32. Verfahren nach Anspruch 25, dadurch gekennzeichnet, daß das große Molekül ein Protein ist.

33. Verfahren nach Anspruch 25, dadurch gekennzeichnet, daß das große Molekül ein Antikörper ist.

34. Verfahren nach Anspruch 25, dadurch gekennzeichnet, daß das große Molekül ein Antigen ist.

**Revendications**

1. Procédé pour influer sur la fixation et/ou l'orientation de grosses molécules sur un substrat, consistant à modifier la chimie de surface dudit substrat en soumettant la surface dudit substrat à un plasma qui est produit à partir d'un matériau choisi dans le groupe comprenant le carbone, l'hydrogène, l'oxygène, l'azote, le soufre, le phosphore, les halogènes et leurs dérivés.

2. Procédé selon la revendication 1, dans lequel ledit plasma est un plasma de décharge, produit à une fréquence d'environ 1 à environ 300 mégahertz, pour une puissance d'environ 5 à environ 1000 watts, sous une pression d'environ 0,13 à 1333 Pa (d'environ 0,001 à environ 10 Torr).

3. Procédé selon les revendications 1 ou 2, dans lequel ledit matériau pour plasma est choisi entre l'oxygène et les composés oxygénés.

4. Procédé selon les revendications 1 ou 2, dans lequel ledit matériau pour plasma est choisi entre l'azote et les composés azotés.

5. Procédé selon les revendications 1 ou 2, dans lequel ledit matériau pour plasma est choisi entre le soufre et les composés soufrés.

6. Procédé selon les revendications 1 ou 2, dans lequel ledit matériau pour plasma est choisi entre le phosphore et les composés phosphorés.

7. Procédé selon les revendications 1 ou 2, dans lequel ledit matériau pour plasma est choisi entre les halogénes et les composés halogénés.

8. Procédé selon les revendications 1 ou 2, dans lequel ledit matériau pour plasma est choisi entre le carbone et les composés carbonés.

9. Procédé selon les revendications 1 ou 2, dans lequel ledit matériau pour plasma est choisi entre l'hydrogène et les composés hydrogénés.

10. Procédé selon la revendication 1, dans lequel ladite grosse molécule est une protéine.

11. Procédé selon la revendication 1, dans lequel ladite grosse molécule est un anticorps.

12. Procédé selon la revendication 1, dans lequel ladite grosse molécule est un antigène.

13. Objet permettant d'influer sur la fixation et/ou l'orientation de grosses molécules par rapport à lui-même, comprenant un objeten polymère dont la chimie de surface a été modifiée en soumettant la surface dudit objet en polymère à un plasma qui est produit à partir d'un matériau choisi dans le groupe comprenant le carbone, l'hydrogène, l'oxygène, l'azote, le soufre, le phosphore, les halogènes et leurs dérivés, dans lequel le plasma est un plasma de décharge produit à une fréquence d'environ 1 à environ 300 mégahertz, pour une puissance d'environ 5 à environ 1000 watts, sous ne pression d'environ 0,13 à environ 1333 Pa (environ 0,001 à environ 10 Torr), caractérisé en ce que le matériau pour plasma est choisi entre l'oxygène et les composés oxygénés.

14. Objet selon la revendication 13, dans lequel ledit matériau pour plasma est choisi entre l'azote et les composés azotés.

15. Objet selon la revendication 13, dans lequel ledit matériau pour plasma est choisi entre le soufre et les composés soufrés.

16. Objet selon la revendication 13, dans lequel ledit matériau pour plasma est choisi entre le phosphore et les composés phosphores.

17. Objet selon la revendication 13, dans lequel ledit matériau pour plasma est choisi entre l'hydrogène et les composés hydrogénés.

18. Objet selon la revendication 13, dans lequel ladite grosse molécule est une protéine.

19. Objet selon la revendication 13, dans lequel ladite grosse molécule est un anticorps.

20. Objet selon la revendication 13, dans lequel ladite grosse molécule est un antigène.

21. Objet permettant d'influer sur la fixation et/ou l'orientation de grosses molécules par rapport à lui-même, comprenant un objet minéral dont la chimie de surface a été modifiée en soumettant la surface dudit objet minéral à un plasma qui est produit à partir d'un matériau choisi dans le groupe comprenant le carbone, l'hydrogène, l'oxygène, l'azote, le soufre, le phosphore, les halogènes et leurs dérivés, dans lequel le plasma est un plasma de décharge produit à une fréquence d'environ 1 à environ 300 mégahertz, pour une puissance d'environ 5 à environ 1000 watts, sous une pression d'environ 0,13 à 1333 Pa (0,001 à environ 10 Torr), caractérisé en ce que la matériau pour plasma est choisi entre l'oxygène et les composés oxygénes.

22. Objet selon la revendication 21, dans lequel ledit matériau pour plasma est choisi entre l'azote et les composés azotés.

23. Objet selon la revendication 21, dans lequel ledit matériau pour plasma est choisi entre le soufre et les composés soufrés.

24. Objet selon la revendication 21, dans lequel ledit matériau pour plasma est choisi entre le phosphore et les composés phosphorés.

25. Procédé pour influer sur la fixation et/ou l'orientation de grosses molécules sur un substrat, consistant à modifier la chimie de surface dudit substrat, par un procédé consistant:

(a) à placer ledit substrat à l'intérieur d'une zone de réaction,

(b) à introduire un matériau dans ladite zone de réaction, ledit matériau convenant à la création d'un plasma,

(c) à soumettre ledit matériau à des conditions de production d'un plasma, de telle sorte que le plasma comprenne un matériau neutre, des ions positifs dudit matériau, des ions négatifs dudit matériau, des électrons et des photons,

(d) à empêcher qu'au moins l'une des constituants dudit plasma n'entre en contact avec la surface dudit substrat,

(e) à mettre en contact la surface dudit substrat avec le reste desdits constituants dudit plasma, et

(f) à former des groupes fonctionnels spécifiques dudit matériau sur la surface dudit substrat organique.

26. Procédé selon la revendication 25, dans lequel ledit plasma est produit par une décharge électrique.

27. Procédé selon les revendications 25 et 26, dans lequel ledit matériau est choisi entre l'oxygène, l'azote, les halogènes, le soufre, leurs dérivés et les mélanges de ceux-ci.

28. Procédé selon les revendications 25 et 26, dans lequel ledit matériau vaporisé est l'oxygène et ses dérivés.

29. Procédé selon les revendications 25 et 26, dans lequel ledit matériau vaporisé est l'azote et ses dérivés.

30. Procédé selon les revendications 25 et 26, dans lequel ledit matériau vaporisé est un halogène et ses dèrivés.

31. Procédé selon les revendications 24 et 26, dans lequel ledit matériau vaporisé est le soufre et ses dérivés.

32. Procédé selon la revendication 25, dans lequel ladite grosse molécule est une protéine.

33. Procédé selon la revendication 25, dans lequel ladite grosse molécule est un anticorps.

34. Procédé selon la revendication 25, dans lequel ladite grosse molécule est un antigène.

FIG. 1·

FIG. 2

FIG. 3

FIG. 4B

FIG. 4A

FIG.5A

FIG.5B

0 104 608

FIG.6A

FIG.6B